(19) Europäisches Patentamt
European Patent Office
Office européen des brevets

(11) Publication number: **0 072 090**
**B1**

(12) **EUROPEAN PATENT SPECIFICATION**

(45) Date of publication of patent specification: **02.04.86**

(51) Int. Cl.$^4$: **C 07 D 501/46**

(21) Application number: **82303019.2**

(22) Date of filing: **11.06.82**

(54) **Process for purifying cephalosporin compounds.**

(30) Priority: **16.06.81 JP 92837/81**

(43) Date of publication of application:
**16.02.83 Bulletin 83/07**

(45) Publication of the grant of the patent:
**02.04.86 Bulletin 86/14**

(84) Designated Contracting States:
**CH DE FR GB LI**

(56) References cited:
**EP-A-0 019 067**
**FR-A-2 394 550**

(73) Proprietor: **AJINOMOTO CO., INC.**
**5-8, Kyobashi 1-chome, Chuo-ku**
**Tokyo 104 (JP)**

(72) Inventor: **Saito, Hideomi**
**No. 143, Aihara**
**Sagamihara-shi Kanagawa-ken (JP)**
Inventor: **Yamamoto, Tomoya**
**No. 2261-4, Kamigo-cho Totsuka-ku**
**Yokohama-shi Kanagawa-ken (JP)**
Inventor: **Nomura, Masayuki**
**No. 2297-2, Okazu-cho Totsuka-ku**
**Yokohama-shi Kanagawa-ken (JP)**
Inventor: **Hachiya, Tomoyoshi**
**No. 2-15-22, Kita**
**Kunitachi-shi Tokyo (JP)**

(74) Representative: **Jones, Michael Raymond et al**
**HASELTINE LAKE & CO. Hazlitt House**
**28 Southampton Buildings Chancery Lane**
**London WC2A 1AT (GB)**

**EP 0 072 090 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for purifying cephalosporin compounds which may be used as antibacterial agents, particularly medicines for treating infective diseases of human beings and animals caused by *Pseudomonsa aeruginosa,* or as intermediates for producing such medicines. More particularly, the present invention relates to a process for purifying a cephalosporin compound (as disclosed in U.S. Patent No. 4217450) to isolate it from impurities by treatment with an absorbing resin.

According to the present invention, there is provided a process for purifying a cephalosporin compound to isolate it from any impurities by treatment with an adsorbing resin, wherein the cephalosporin compound has the following general formula:

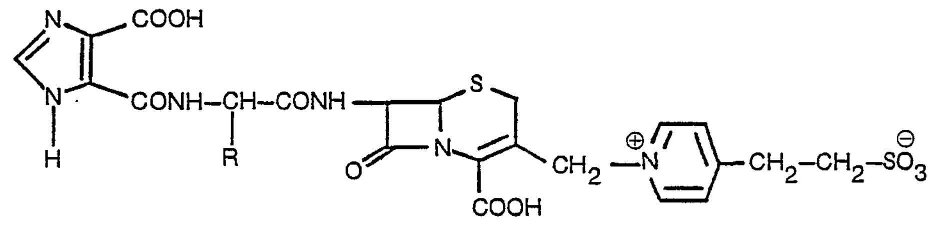

(1)

in which R is a phenyl group or a p-hydroxyphenyl group; and wherein the adsorbing resin is a resin of a high molecular material made of polyacrylate and having a hydrophilic fine network structure; the process comprising adjusting the pH of an aqueous solution containing the cephalosporin compound to be purified in a concentration of 1 to 10% to 1.0 to 3.5, removing any precipitate thus formed, and subjecting the resulting aqueous solution to treatment with the adsorbing resin.

The cephalosporin compounds represented by the foregoing general formula can be produced according to various synthesizing processes, for example by reacting a compound represented by the general formula:

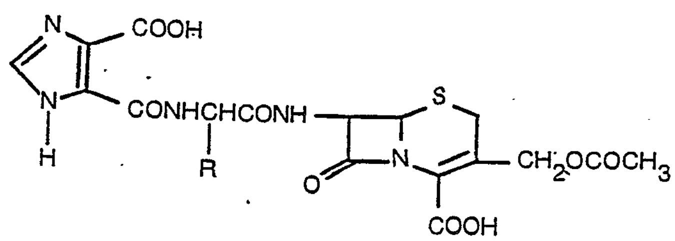

(2)

(wherein R is as defined above) with 4-pyridineethanesulphonic acid in an aqueous solution in the presence of sodium iodide, as disclosed in EP—A—60028 (European Patent Application No. 82300650.7).

However, complete removal of impurities (such as colouring impurities whose production is attributable to synthesizing processes from the cephalosporin compounds) has been difficult even by purification techniques such as, for example, reprecipitation or recrystallization. Thus, for instance, after the reaction is conducted in the presence of sodium iodide, an organic solvent which dissolves sodium iodide but does not dissolve the desired end product, such as methanol, ethanol, propanol, acetone or methyl ethyl ketone, may be added to the reaction solution (from which any solids are removed) to separate the end product as a solid product. (In this case, an alternative is to add an aqueous solution containing the end product to the above-mentioned organic solvent). The end product can be separated by repeating this procedure several times. However, the resulting product is still inadequate as a safe product with no biological toxicity. Thus a need has remained for a process for purifying cephalosporin compounds with high purity in a high yield.

Preferred ways of carryng out the process of the present invention will now be described.

A reaction solution containing the desired cephalosporin compound end product is added to a 3- to 7-fold volume of organic solvent cooled to 5 to 20°C such as methanol, ethanol, propanol, acetone or methyl ethyl ketone, and any precipitate which is formed is removed by filtration or centrifuging. With reactions using sodium iodide, sodium iodide can be removed to a mother liquor side. The thus obtained precipitate is dissolved in water, and again a 3- to 7-fold volume of the above-mentioned organic solvent is added thereto to form a precipitate, followed by separating the thus formed precipitate to obtain a crude product. Repetition of the same procedure yields a crude product with a higher purity.

This crude product is dissolved in water and, after adjusting the concentration to 1—10%, preferably 2—4%, an inorganic acid such as hydrochloric acid, sulphuric acid or nitric acid or an organic acid such as formic acid, acetic acid or oxalic acid is added thereto to adjust the pH to 1.0 to 3.5, preferably 1.5 to 2.5, for precipitating impurities. The temperature in this procedure is suitably 10 to 35°C. The precipitate thus

formed is removed by filtration or centrifuging, and the resulting mother liquor is concentrated to 10 to 15% at 20 to 40°C, and added to a 3- to 7-fold volume of the organic solvent as mentioned above at a temperature of 5 to 20°C to precipitate the end product, followed by separating it by filtration or centrifuging. Water is added to the thus separated precipitate to make a 5—20% aqueous solution, preferably adjusted to pH 3 ~ 4 in view of good recovery of the desired end product and good separation of impurities, and impurities contained therein are removed using a 0.1- to 10-fold volume of a high molecular material having hydrophilic fine network structure, such as "Amberlite® XAD—7 or XAD—8" made by Rohm & Haas Co. or "Diaion® HP—IMG, HP—2MG or HP—3MG" made by Mitsubishi Chemical Industries Ltd. according to a batchwise process or chromatography.

The resulting aqueous solution containing the end product is concentrated to a concentration of 10 to 15% at a temperature of 20 to 30°C, and a 3- to 7-fold volume of organic solvent as indicated above cooled to 20°C is added thereto to precipitate the desired end product. After being separated by filtration or centrifuging, the precipitate is dried in a conventional manner to obtain the highly purified end product. The thus purified cephalosporin compounds are stable products.

The present invention will now be described in yet more detail, and by way of specific illustration, by reference to the following examples.

Example 1

7.13 Grams (13.1 mmol) of 7β-[D-(−)-α-(4-carboxyimidazole-5-carboxamido)-phenylacetamido]-cephalosporanic acid and 4.9 g (26.3 mmol) of 4-pyridineethanesulphonic acid were suspended in 30 ml of water, and the pH of the solution was adjusted to 6.5 with 2 *N* sodium hydroxide to dissolve them. After adding thereto 87.5 g of sodium iodide, the mixture was heated to 65°C for 70 minutes to react, under stirring. After being cooled, the reaction solution was added dropwise to 330 ml of acetone under ice-cooling and stirring. After cooling the mixture overnight, a solid product which formed was collected by filtration, again dissolved in 30 ml of water, and added dropwise to 150 ml of acetone to precipitate a solid product. The solid product thus formed was collected by filtration, dissolved in 30 ml of water, and added dropwise to 200 ml of ethanol under ice-cooling and stirring. After cooling overnight, a solid product which formed was collected and dried to obtain a crude product containing 7.0 g (70.0% in purity) of sodium 7β-[D-(−)-α-(4-carboxyimidazole-5-carboxamido)-phenylacetamido]-3-(4-β-sulphoethylpyridinium)methyl-3-cephem-4-carboxylate represented by the following chemical formula:

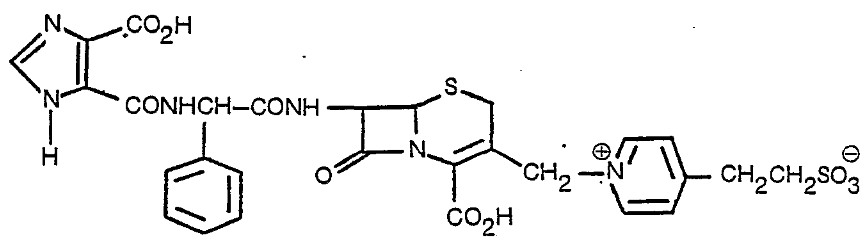

Then, 13.7 g of this crude product was dissolved in 400 ml of 30°C water, and 6 *N* hydrochloric acid was added thereto to adjust the pH to 2. After continuing the stirring for 30 minutes, a precipitate which formed was removed by centrifuging, and the mother liquor was concentrated at 30°C under reduced pressure to a quantity of 80 g. To this concentrate were added 400 ml of ethanol under stirring and, after being cooled to 10°C, the solution was allowed to stand for one hour. A precipitate which formed was collected by filtration under reduced pressure, and dried in vacuo at 40°C to obtain 10.1 g of powder containing 8.6 g of the end product.

Further, water was added to 10.1 g of this powder to make a 100 ml solution, and then 25 ml of a non-ionic absorbing resin, "Amberlite XAD—7", was added thereto. After stirring for 30 minutes at room temperature, the resin was removed by filtration, and the resin was washed with 50 ml of water. The mother liquor and the washing were combined and concentrated at 30°C to obtain 70 g of a concentrate. Then 350 ml of ethanol were added to this concentrate under stirring, and the resulting solution was cooled to 10°C and allowed to stand for one hour. A precipitate which formed was separated by filtration under reduced pressure and dried in vacuo to obtain 7.3 g of the end product (yield: 73%). Reverse phase thin layer chromatography (TLC) of 20 μg of the product gave a mono spot. In contrast, reverse phase TLC of 20 μg of the aforesaid crude product gave four spots.

Example 2

13.7 Grams of a crude product (containing 9.6 g of the desired end product) prepared according to the procedure described in Example 1 were dissolved in 500 ml of 10°C water, and the pH of the solution was adjusted to 2 by adding thereto 6 *N* sulphuric acid. After continuing stirring for 30 minutes, a precipitate which formed was removed by centrifuging, and the mother liquor was concentrated at 30°C under reduced pressure to a quantity of 80 g. To this concentrate were added, under stirring, 400 ml of ethanol. After being cooled to 10°C, the solution was allowed to stand for one hour, and the precipitate which

formed was separated by filtration under reduced pressure and dried in vacuo at 40°C to obtain 9.6 g of powder containing 7.7 g of the end product.

Further, water was added to 9.6 g of this powder to make a 76 ml solution, and this solution was passed through a resin tower containing 100 ml of a non-ionic absorbing resin, "Amberlite XAD—7", to collect fractions containing the end product, followed by concentrating them at 30°C to obtain 60 g of a concentrate. Then 300 ml of ethanol were added to this concentrate under stirring. After being cooled at 10°C, the solution was allowed to stand for one hour, and a precipitate which formed was collected by filtration under reduced pressure and dried in vacuo at 40°C to obtain 6.9 g of the end product (yield: 72%). Reverse phase TLC of 20 μg of this substance gave a mono spot.

Example 3

13.7 Grams of a crude product (containing 9.6 g of the end product) prepared according to the procedure described in Example 1 were dissolved in 400 ml of 30°C water, and the pH of the solution was adjusted to 2.5 by adding thereto 6 *N* hydrochloric acid. After continuing stirring for 30 minutes, a precipitate which formed was removed by filtration, and the mother liquor was concentrated to a quantity of 80 g at 30°C under reduced pressure. Then 400 ml of ethanol were added, under stirring, to this concentrate. After being cooled to 10°C, the solution was allowed to stand for 1 hour, and a precipitate which formed was separated by filtration under reduced pressure, and dried in vacuo at 40°C to obtain 10.1 g of powder containing 8.6 g of the end product.

Further, water was added to 9.6 g of this powder to make a 80 ml solution, and the resulting solution was passed through a resin tower retaining 100 ml of a non-ionic absorbing resin, "Diaion HP—3MG" (manufactured by Mitsubishi Chemical Industries Ltd.), to collect fractions containing the end product. The fractions were then concentrated at 30°C to obtain a 70 g concentrate. Then 350 ml of ethanol were added to this concentrate under stirring. After being cooled to 10°C, the solution was allowed to stand for one hour, and a precipitate which formed was separated by filtration under reduced pressure, and dried at 40°C to obtain 7.7 g of the end product (yield: 80%). Reverse phase TLC of 20 μg of this substance gave a mono spot.

**Claims**

1. A process for purifying a cephalosporin compound to isolate it from any impurities by treatment with an adsorbing resin, wherein the cephalosporin compound has the following general formula:

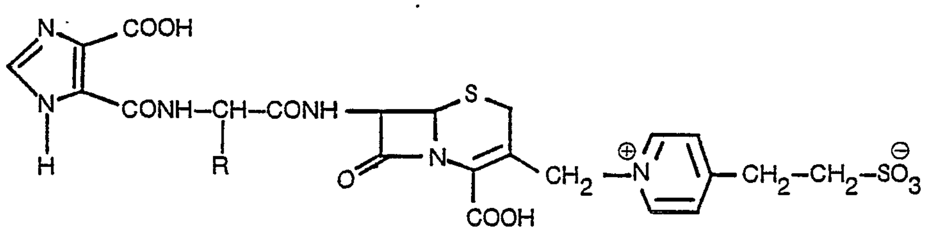

(1)

in which R is a pheny group or a p-hydroxyphenyl group; and wherein the adsorbing resin is a resin of a high molecular material made of polyacrylate and having a hydrophilic fine network structure; the process comprising adjusting the pH of an aqueous solution containing the cephalosporin compound to be purified in a concentration of 1 to 10% to 1.0 to 3.5, removing any precipitate thus formed, and subjecting the resulting aqueous solution to treatment with the adsorbing resin.

2. A process according to claim 1, wherein, prior to the production of the aqueous solution containing the cephalosporin compound, a solution containing the cephalosporin compound is mixed with an organic solvent to leave impurities in solution and to precipitate the cephalosporin compound.

3. A process according to claim 1 or 2, wherein the concentration of the aqueous solution containing the cephalosporin compound is from 2 to 4%, the pH is adjusted to 1.5 to 2.5 and the temperature during pH adjustment is from 25 to 30°C.

4. A process according to claim 3, wherein, after removing the precipitate produced by the pH adjustment, the cephalosporin compound is separated by addition of an organic solvent, after which the separated cephalosporin compound in aqueous solution is treated with the adsorbing resin.

5. A process according to any preceding claim, wherein the adsorbing resin is Amberlite® XAD—7 or XAD—8, or Diaion® HP—1MG, HP—2MG or HP—3MG.

**Patentansprüche**

1. Verfahren zur Reinigung einer Cephalosporin-Verbindung durch Behandlung mit einem adsorbierenden Harz, um sie von Verunreinigungen zu isolieren, wobei die Cephalosporin-Verbindung die folgende allgemeine Formel hat:

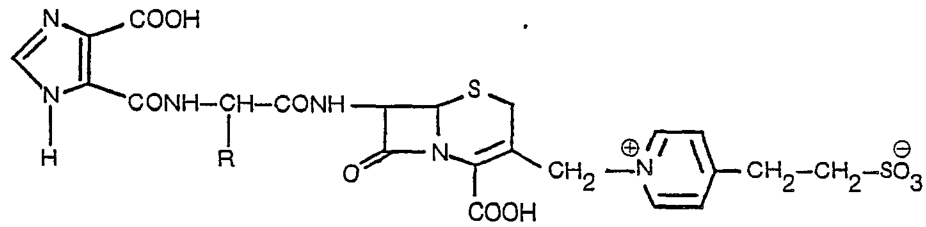

worin R eine Phenylgruppe oder eine p-Hydroxyphenylgruppe ist, und wobei das adsorbierende Harz ein Harz aus einem aus Polyacrylat gebildeten hochmolekularen Material ist, das eine hydrophile feine Netzstruktur hat, dadurch gekennzeichnet, daß der pH-Wert einer wässrigen Lösung, welche die zu reinigende Cephalosporin-Verbindung in einer Konzentration von 1 bis 10% enthält, auf 1,0 bis 3,5 eingestellt wird, ein auf diese Weise gebildeter Niederschlag abgetrennt und die gebildete wässrige Lösung der Behandlung mit dem adsorbierenden Harz unterworfen wird.

2. Verfahren nach Anspruch 1, bei dem vor der Herstellung der die Cephalosporin-Verbindung enthaltenden wässrigen Lösung eine Lösung, welche die Cephalosporin-Verbindung enthält, mit einem organischen Lösungsmittel vermischt wird, un die Verunreinigungen in Lösung zu halten und die Cephalosporin-Verbindung auszufällen.

3. Verfahren nach Anspruch 1 oder 2, bei dem die Konzentration der die Cephalosporin-Verbindung enthaltenden wässrigen Lösung 2 bis 4% beträgt, der pH-Wert auf 1,5 bis 2,5-eingestellt wird und die Temperatur während der pH-Einstellung bei 25 bis 30°C gehalten wird.

4. Verfahren nach Anspruch 3, bei dem nach dem Entfernen des durch die pH-Einstellung gebildeten Niederschlags die Cephalosporin-Verbindung durch Zugabe eines organischen Lösungsmittels abgetrennt wird, wonach die abgetrennte Cephalosporin-Verbindung in wässriger Lösung mit dem adsorbierenden Harz behandelt wird.

5. Verfahren nach einem der vorhergehenden Patentansprüche, bei dem das adsorbierende Harz Amberlite® XAD—7 oder XAD—8 oder Diaion® HP—1MG, HP—2MG oder HP—3MG ist.

**Revendications**

1. Un procédé pour la purification d'un dérivé de céphalosporine afin de l'isoler de toute impureté par traitement avec une résine adsorbante dans lequel le dérivé de céphalosporine répond à la formule générale suivante:

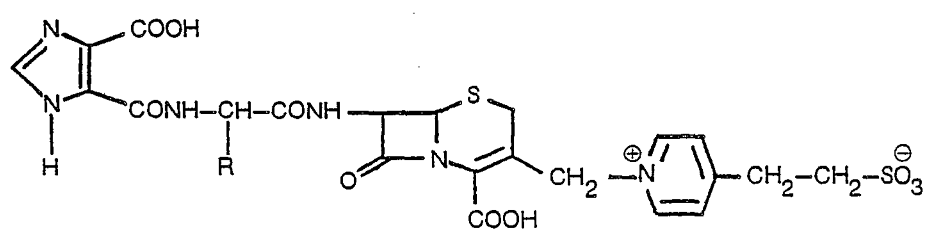

dans laquelle R est un groupe phényle ou un group p-hydroxyphényle; et où la résine adsorbante est une résine d'une matière de poids moléculaire élevé faite de polyacrylate et ayant une structure réticulée fine hydrophile; le procédé comprenant l'ajustement du pH d'une solution aqueuse contenant le dérivé de céphalosporine à purifier à une concentration de 1 à 10% entre, 1,0 et 3,5, l'élimination de tout précipité ainsi formé et le traitement de la solution aqueuse obtenue avec la résine adsorbante.

2. Un procédé selon la revendication 1 dans lequel avant la production de la solution aqueuse contenant le dérivé de céphalosporine, on mélange une solution contenant le dérivé de céphalosporine avec un solvant organique pour laisser les impuretés en solution et pour précipiter le dérivé de céphalosporine.

3. Un procédé selon la revendication 1 ou 2 dans lequel la concentration de la solution aqueuse contenant le dérivé de céhalosporine est de 2 à 4%, le pH est ajusté entre 1,5 et 2,5 et la température pendant l'ajustement du pH est de 25 à 30°C.

4. Un procédé selon la revendication 3 dans lequel, après élimination du précipité produit par l'ajustement du pH, on sépare le dérivé de céphalosporine par addition d'un solvant organique puis on traite le dérivé de céphalosporine séparé en solution aqueuse avec la résine adsorbante.

5. Un procédé selon l'une quelconque des revendications précédentes dans lequel la résine adsorbante est l'Amberlite® XAD—7 ou XAD—8 ou le Diaion® HP—1MG, HP—2MG ou HP—3MG.